(19) Europäisches Patentamt
European Patent Office
Office européen des brevets

(11) Veröffentlichungsnummer : **0 034 279**
**B1**

(12) # EUROPÄISCHE PATENTSCHRIFT

(45) Veröffentlichungstag der Patentschrift : **22.06.83**

(21) Anmeldenummer : **81100611.3**

(22) Anmeldetag : **28.01.81**

(51) Int. Cl.$^3$ : **C 07 C143/68**, C 07 D235/08, C 07 D263/56, C 07 D263/60, C 07 D277/64, C 07 D277/84, C 07 D293/12, C 07 D403/12, C 07 D417/04, C 09 B 23/02

(54) **Sulfoalkylierungsmittel, Verfahren zu ihrer Herstellung und ihre Verwendung zur Herstellung von Sulfoalkylquartärsalzen.**

(30) Priorität : **08.02.80 DE 3004692**
**19.03.80 DE 3010427**

(43) Veröffentlichungstag der Anmeldung : **26.08.81 Patentblatt 81/34**

(45) Bekanntmachung des Hinweises auf die Patenterteilung : **22.06.83 Patentblatt 83/25**

(84) Benannte Vertragsstaaten : **BE CH DE FR GB IT LI**

(56) Entgegenhaltungen :
**DE A 2 803 493**
**CHEMISCHE BERICHTE, Band 85, Nr. 2, 1952 Weinheim-Bergstrasse G. MANECKE « Notiz über die Einwirkung von Sulfurylchlorid auf n-propylalkohol » Seite 161, Formel V**

(73) Patentinhaber : **AGFA-GEVAERT Aktiengesellschaft**
**D-5090 Leverkusen 1 (DE)**

(72) Erfinder : **Kampfer, Helmut, Dr.**
**Roggendorfstrasse 63**
**D-5000 Köln 80 (DE)**
Erfinder : **Wendisch, Detlef, Dr.**
**August-Kierspel-Strasse 145**
**D-5060 Bergisch Gladbach 2 (DE)**
Erfinder : **Hase, Marie, Dr.**
**Im Birkelshof 5**
**D-5060 Bergisch Gladbach 2 (DE)**
Erfinder : **Glass, Max**
**Jasminweg 18**
**D-5000 Köln 80 (DE)**

**EP 0 034 279 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

# Sulfoalkylierungsmittel, Verfahren zu ihrer Herstellung und ihre Verwendung zur Herstellung von Sulfoalkylquartärsalzen

Die Erfindung betrifft neue Sulfoalkylierungsmittel der vermuteten Struktur der Formel I, Verfahren zu ihrer Herstellung und ihre Verwendung zur Herstellung von Sulfoalkylquartärsalzen tertiärer Amine, insbesondere stickstoffhaltiger heterocyclischer Basen.

Sulfonsäureester werden im allgemeinen aus Sulfonsäurehalogeniden und entsprechenden Alkoholen hergestellt. Sie finden als ausgezeichnete Alkylierungsmittel Anwendung.

Eine spezielle Gruppe von Sulfonsäureestern leitet sich von Hydroxyalkansulfonsäuren durch intramolekulare Esterbildung ab. Bekannt sind die als Sulfonylide bezeichneten cyclischen inneren Ester, von denen die Sultone als Sulfoalkylierungsmittel erhebliche technische Bedeutung erlangt haben. Unter den Sultonen sind dies inbesondere die 5-gliedrigen 1,3-Propansultone (= 1,2-Oxathiolan-2,2-dioxide) wegen ihrer hohen Reaktionsfähigkeit gegenüber alkylierbaren Verbindungen. Sie lassen sich leicht aus 3-Hydroxy- oder 3-Halogenpropansulfonsäuren durch thermischen Ringschluß unter gleichzeitiger Destillation im Vakuum gewinnen. Nachteilig an den Sultonen ist deren physiologische Wirkung und die damit verbundenen Probleme bei der Lagerung, beim Transport und sonstigem Umgang.

Daher hat es nicht an Versuchen gefehlt, Sultone durch andere, unbedenkliche Sulfoalkylierungsmittel zu ersetzen.

So sind Hydroxyalkansulfonsäuren und ihre Salze als Quaternierungsmittel für heterocyclische tertiäre Amine beschrieben worden. Ferner wurden für den gleichen Verwendungszweck O-Sulfoalkylimidoester und Uroniumsalze empfohlen. Diese Verfahren sind jedoch auf die Alkylierung von tertiären Basen beschränkt und somit weniger allgemein anwendbar als die Alkylierung mit Sultonen. Außerdem sind Imidoester und Uroniumsalze realtiv teuer in der Herstellung.

Aus diesen Gründen bestand erhebliches Interesse an billigen, physiologisch unbedenklichen Ersatzstoffen für Sultone, die die genannten Nachteile nicht besitzen.

Ein Gegenstand der Erfindung sind Sulfoalkylierungsmittel der vermuteten Struktur der folgenden Formel I (intermolekulare Ester der Hydroxyalkansulfonsäuren oder « dimere Hydroxyalkansulfonsäuren ») :

$$HO{-}(\underset{R}{CH})_n SO_2{-}O{-}(\underset{R}{CH})_n{-}SO_3X \qquad (I)$$

worin bedeuten

X = H oder ein Kation, vorzugsweise ein Alkalimetallion oder Ammoniumion,

n = 3 oder 4, vorzugsweise 3,

und die Reste R in jeder Gruppe $-\underset{R}{CH}-$ unabhängig voneinander H oder Alkyl mit 1 bis 4 C-Atomen, insbesondere Methyl, wobei die Alkylgruppe gegebenenfalls substituiert sein kann mit Halogen wie Fluor oder Chlor.

Besonders geeignet sind Verbindungen der folgenden Formel II :

$$HO(CH_2)_2{-}\underset{R}{CH}{-}SO_2{-}O(CH_2)_2{-}\underset{R}{CH}{-}SO_3X \qquad (II)$$

worin R und X die oben angegebene Bedeutung haben.

Ein weiterer Gegenstand der Erfindung ist ein Verfahren zur Herstellung der « Ester » I durch Erwärmen der entsprechenden Hydroxyalkansulfonsäuren, vorzugsweise solcher der Formel III, oder der entsprechenden Alkansultone, vorzugsweise solcher der Formel IV, worin R die oben angegebene Bedeutung hat, mit der 1- bis 4-fachen molaren Menge Wasser auf Temperaturen zwischen 80 und 180 °C, vorzugsweise zwischen 120 und 150 °C.

$$HO{-}CH_2{-}CH_2{-}\underset{R}{CH}{-}SO_3H \qquad (III)$$

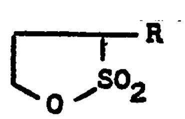

(IV)

Die erfindungsgemäßen Sulfoalkylierungsmittel sind hygroskopische Substanzen, die mit 1-1,5 Mol Wasser kristallisieren. In verdünnter wäßriger Lösung hydrolysieren sie unter Bildung der Hydroxyalkansulfonsäuren. Durch Erhitzen unter dehydratisierenden Bedingungen wie Abdampfen von $H_2O$, azeotrope Destillation, Gegenwart wasserbindender Mittel tritt leicht Cyclisierung zum entsprechenden Sulton ein. Die $^{13}C$-shifts der NMR-Spektren lassen vermuten, daß die erfindungsgemäßen Sulfoalkylierungsmittel in der Struktur der Formel I vorliegen.

Ein niederes Homologes der erfindungsgemäßen « dimeren Hydroxyalkansulfonsäuren » ist in dem Artikel von G. Manecke, « Notiz über die Einwirkung von Sulfurylchlorid auf n-Propylalkohol » in Chem.

Ber. *85*, 160 (1952) als Verbindung V erwähnt. Diese Verbindung V ist ein nicht isoliertes, hypothetisches Zwischenprodukt bei der Hydrolyse des « Sulfonylids » IV. Im Gegensatz zu dem ringförmigen Sulfonylid, dessen sulfoalkylierende Wirkung durch Umsetzung mit Pyridin nachgewiesen worden ist, sind von der Verbindung V keine sulfoalkylierenden Eigenschaften zu erwarten.

Die erfindungsgemäßen Sulfoalkylierungsmittel können anstelle der üblichen Sultone, z. B. anstelle von 1,3-Propansultonen, zur Sulfoalkylierung von dazu befähigten Verbindungen mit funktionellen O-, S-, N- bzw. P-enthaltenden Gruppen verwendet werden. Insbesondere durch die einfache Herstellbarkeit und ihre leichte Recyclisierung zu den Sultonen können die Sulfoalkylierungsmittel als umweltfreundliche « Transportform für Sultone » verwendet werden.

Recyclisierung und folgende Sulfoalkylierung können in abgeschlossenen Reaktionsgefäßen in einem Arbeitsgang durchgeführt werden, wodurch die Freisetzung schädlicher Sultone vermieden wird. Die Herstellung der Als Ausgangsmaterial für die Sulfoalkylierungsmittel in Frage kommenden 3-Hydroxyalkansulfonsäuren ist in der DE-OS 2 803 493 und die der entsprechenden Sultone in der Monographie D. S. BRESLOW, H. SKOLNIK « Multi-Sulfur and Sulfur and Oxygen Five- and Six-Membered Heterocycles », Chapter 4, S. 78 ff., Intersci. Publ. 1966, angegeben.

Die Herstellung einiger der erfindungsgemäßen Sulfoalkylierungsmittel wird im folgenden beschrieben. Andere Verbindungen der Formel I werden in analoger Weise hergestellt.

## Beispiel 1

3-Hydroxypropansulfonsäure-(3-sulfopropyl)-ester (« Ester 1 »)

a) 1 Mol 1,3-Propansulton wird mit 3 Mol Wasser 2 Stunden auf 137 °C unter Rückfluß erhitzt. Dann wird das Reaktionsprodukt am Rotationsverdampfer bei 40 °C und 0,4 mbar vom überschüssigen Wasser befreit. Man erhält den Ester als zähes, beim Stehen kristallisierendes Öl, das nach Titration noch 9 % Wasser enthält und nach dem $^{13}C$-NMR-Spektrum frei von 1,3-Propansulton ist.

b) 1 Mol 3-Hydroxypropansulfonsäure wird mit 3 Mol Wasser 2 Stunden auf 137 °C erhitzt. Die weitere Behandlung wie unter a) angegeben, ergibt ein kristallisierendes Öl mit 8 % Wassergehalt, das ebenfalls frei von 1,3-Propansulton ist.

Recyclisierung :

Durch 4-stündiges Erhitzen von « Ester 1 » auf 140 °C (Badtemperatur) und 27 mbar unter Abdestillation entstehenden Wassers wird 1,3-Propansulton in 88 %iger Ausbeute zurückerhalten.

## Beispiel 2

4-Hydroxybutan-2-sulfonsäure-(3-sulfobutyl)-ester (« Ester 2 »)

4-Hydroxybutan-2-sulfonsäure mit einem Wassergehalt von 14,7 % wird 2 Stunden in einem Ölbad von 170 °C Badtemperatur (bei einer Temperatur des Reaktionsgutes von 144-146 °C) erhitzt. Danach wurde das abgespaltene Wasser bei 40 °C und 0,7 mbar unter Verwendung einer auf – 38 °C gekühlten Vorlage abgedampft.

Die $^{13}C$-NMR-spektroskopisch bestimmte Ausbeute betrug 96,6 %.

Die Charakterisierung der erfindungsgemäßen Verbindungen gemäß Beispielen 1 und 2 erfolgt durch NMR-spektroskopische Daten (im Vergleich zu den entsprechenden Hydroxyalkansulfonsäuren (5) und (6) und 1,3-Propansultonen (3) und (4)).

Tabelle 1 zeigt die $^{13}C$-Shifts des 3-Hydroxypropansulfonsäure(3-sulfopropyl)-esters (1), gemessen in $D_2O$.

$$HO-\overset{3}{C}H_2-\overset{2}{C}H_2-\overset{1}{C}H_2-SO_2-O-\overset{1'}{C}H_2-\overset{2'}{C}H_2-\overset{3'}{C}H_2-SO_3H \qquad (1)$$

Tabelle 1 : $^{13}C$-Shifts (ppm, rel. zu TMS = 0) von (1) in $D_2O$

| C-Atom | $\delta$ (ppm) |
|---|---|
| 1 | 48,8 |
| 2 | 27,8 |
| 3 | 61,1 |
| 1' | 69,5 |
| 2' | 25,2 |
| 3' | 48,8 |

Tabelle 2 enthält die $^{13}C$-Shifts des 4-Hydroxybutan-2-sulfonsäure(3-sulfobutyl)-esters (2) in $D_2O$.

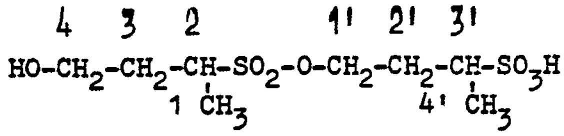
(2)

Tabelle 2 : $^{13}C$-Shifts (ppm, rel. zu TSM = 0) von (2) in $D_2O$

| C-Atom | δ (ppm) |
|---|---|
| 1 | 15,4 |
| 2 | 53,6* |
| 3 | 34,4 |
| 4 | 60,0 |
| 1' | 68,5 |
| 2' | 31,7 |
| 3' | 53,5* |
| 4' | 15,4 |

Die durch Sternchen (*) gekennzeichneten Signale können je nach Art des Experiments auch zusammenfallen.

Die $^{13}C$-Daten von (1) und (2) unterscheiden sich deutlich von denen von (3) und (4).

Tabelle 3 zeigt zunächst die $^{13}C$-Shifts des 1,3-Propansultons (3) in $D_2O$, während Tabelle 4 diejenigen des 3-Methyl-1,3-propansultons (4) enthält.

Die Tabellen 5 und 6 informieren über die $^{13}C$-Daten der 4-Hydroxypropansulfonsäure (5) und der 4-Hydroxybutan-2-sulfonsäure (6).

Tabelle 3 : $^{13}C$-Shifts (ppm, rel. zu TMS = 0) von (3) in $D_2O$

| C-Atom | δ (ppm) |
|---|---|
| 1 | 72,3 |
| 2 | 24,4 |
| 3 | 45,3 |

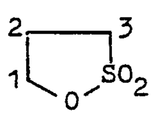
(3)

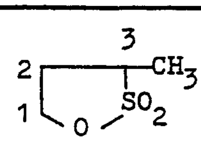
(4)

Tabelle 4 : $^{13}C$-Shifts (ppm, rel. zu TSM = 0) von (4) in $D_2O$

| C-Atom | δ (ppm) |
|---|---|
| 1 | 70,3 |
| 2 | 31,4 |
| 3 | 52,3 |
| $CH_3$ an 3 | 13,3 |

3 2 1

$HO{-}CH_2{-}CH_2{-}CH_2{-}SO_3H$ (5)

4 3 2

$HO{-}CH_2{-}CH_2{-}CH{-}SO_3H$ (6)

1 $CH_3$

Tabelle 5 : $^{13}C$-Shifts (ppm, rel. zu TMS = 0) von (5) in $D_2O$

| C-Atom | δ (ppm) |
|---|---|
| 1 | 48,9 |
| 2 | 27,8 |
| 3 | 61,2 |

Tabelle 6 : $^{13}C$-Shifts (ppm, rel. zu TSM = 0) von (6) in $D_2O$

| C-Atom | δ (ppm) |
|---|---|
| 1 | 15,4 |
| 2 | 53,4 |
| 3 | 34,4 |
| 4 | 60,0 |

Alle Messungen wurden bei 25,2 MHz auf dem Spektrometer XL-100-15" der Firma VARIAN ASSOCIATES, Palo Alto, Calif./USA, im PFT-Betrieb unter Proton-Rauschentkopplung durchgeführt. $D_2O$ diente als Solvens und $^2H$-Lock. Dioxan wurde als interner Standard ($\delta = 67{,}4$ ppm, rel. zu TMS = 0) verwendet. Die Genauigkeit der shift-Messungen beträgt $\pm 0{,}1$ ppm.

Die erfindungsgemäßen Sulfoalkylierungsmittel können beispielsweise zur Sulfoalkylierung von gegebenenfalls alkylierten oder arylierten Aminen verwendet werden, wobei wertvolle Zwischenprodukte für Textilhilfsmittel erhalten werden. Auch zur Gewinnung von Netzmitteln, z. B. durch Umsetzung mit langkettigen Alkoholen, lassen sich die Ester einsetzen. Hochmolekulare Stoffe wie Cellulose oder Eiweiß können durch die Behandlung mit den Estern hydrophile Eigenschaften erhalten. Besonders geeignet sind die erfindungsgemäßen Sulfoalkylierungsmittel zur Herstellung von Sulfoalkylquartärsalzen tertiärer Amine, insbesondere stickstoffhaltiger heterocyclischer Basen.

Derartige Quartärsalze, die positiv und negativ geladene, durch kovalente Bindungen miteinander verbundene Gruppen enthalten, werden auch als Betaine bezeichnet. Sie spielen in einer Reihe von technischen Prozessen eine wichtige Rolle. Dabei werden diese Betaine entweder als solche direkt verwendet, z. B. in der Galvanotechnik, oder als Zwischenprodukte weiteren Umsetzungen unterworfen. Zur Verwendung der Sulfoalkylbetaine als Zwischenprodukte ist es häufig technisch vorteilhaft, diese nicht erst zu isolieren, sondern die weitere Umsetzung in einem Arbeitsgang an deren Herstellung anzuschließen. Als Zwischenprodukte spielen die Sulfoalkylbetaine beispielsweise eine bedeutende Rolle für die Synthese der als spektrale Sensibilisierungsfarbstoffe für lichtempfindliche Materialien, insbesondere für fotografische Silberhalogenidemulsionen, verwendeten Polymethinfarbstoffe.

Verfahren zur Herstellung von Sulfoalkylquartärsalzen tertiärer Amine sind schon lange bekannt. Man setzt die tertiäre Base mit einem Sulfoalkylierungsmittel, für gewöhnlich bei erhöhter Temperatur, um. Als Sulfoalkylierungsmittel sind beschrieben u. a. Halogenalkansulfonsäuren, z. B. 2-Bromethansulfonsäure in US 2 503 776, Na-Iodethansulfonat in BE 669 308, Na-Iodbutansulfonat in US 2 912 329 oder 3-Chlor-2-hydroxypropansulfonsäure in DT-AS 1 177 482. Nachteilig an diesen Sulfoalkylierungsmitteln ist der bei Verwendung der Freien Sulfonsäuren zum Abfangen des entstehenden Halogenwasserstoffs erforderliche Überschuß an tertiärer Base. Ferner sind Sultone als Sulfoalkylierungsmittel bekannt, so sind Propan-, Butan- und Isopentansulton in DT-PS 929 080 beschrieben, Propensulton in DT-AS 1 447 579 oder 2-Chlorpropansulton in GB 1 090 626. Nachteilig an den Sultonen ist deren z. T. schädliche physiologische Wirkung, so daß ihre Verwendung zum Umweltrisiko und zum Sicherheitsrisiko für die damit umgehenden Personen werden kann.

In neuerer Zeit sind Sulfoalkylierungsmittel beschrieben worden, die die Verwendung cancerogener

Sultone vermeiden. So werden in DT-OS 2 825 246 und in Research Disclosure 16374/1977 Hydroxyalkansulfonsäuren und ihre Salze als Quaternierungsmittel beschrieben. Nachteilig sind die bei der Reaktion entstehenden erheblichen Wassermengen, die die Quaternierung behindern und z. T. zu niedrigen Ausbeuten führen bzw. durch azeotrope Destillation mit geeigneten Lösungsmitteln entfernt werden müssen.

Ferner wurden 0-Sulfoalkylimidoester in Research Disclosure 18040/1979 und damit verwandte 0-Sulfoalkylisouroniumbetaine in der DE-OS 2 909 200 beschrieben. Auch diese neuen Quarternierungsmittel sind mit einer Reihe von Nachteilen behaftet. So ist ihre Herstellung durch Verwendung von Carbodiimiden, Säurenitrilen bzw. Dialkylharnstoffen relativ teuer. Ihre thermische Stabilität ist begrenzt. Die teilweise hohe Schmelzpunkte zeigenden Verbindungen zwingen zur Verwendung von Lösungsmitteln als Reaktionsmedium.

Ein weiterer Gegenstand der Erfindung besteht somit in der Verwendung der erfindungsgemäßen Sulfoalkylierungsmittel bei der Umsetzung mit quaternierbaren tertiären Aminen zur Herstellung von Sulfoalkylquartärsalzen.

Die Umsetzung erfolgt bei erhöhter Temperatur, z. B. bei einer Temperatur zwischen 80 und 250 °C, vorzugsweise bei einer Temperatur zwischen 140 und 200 °C. Im allgemeinen läuft die Reaktion innerhalb des zuletzt genannten Temperaturbereiches glatt ab. Es kann jedoch, etwa bedingt durch die Natur eines verwendeten Lösungsmittels, auch außerhalb dieses Temperaturbereiches gearbeitet werden.

An tertiären Aminen sind geeignet prinzipiell alle Derivate des Ammoniak ($NH_3$), in denen jedes der drei Wasserstoffatome substituiert ist, z. B. durch ein Kohlenstoffatom eines Alkyl- oder Arylrestes oder durch ein Kohlenstoffatom oder ein heteroatom eines heterocyclischen Ringes, wobei insbesondere das Stickstoffatom des tertiären Amins in den heterocyclischen Ring einbezogen sein kann. So sind besonders bevorzugt geeignet heterocyclische Basen der allgemeinen Formel V

```
N=(CH-CH)=)mC-Y
:           :
:....Z......:
```
(V)

worin Z die zur Ergänzung einer heterocyclischen Gruppe mit mindestens einem 5- oder 6-gliedrigen heterocyclischen Ring erforderlichen Glieder bedeuten ; der Heteroring kann dabei ankondensierte Benzol-, Naphthalin- oder auch heterocyclische Ringe enthalten, die auch weiter substituiert sein können ; in Frage kommen die aus der Klasse der Cyaninfarbstoffe bekannten Heterocyclen, wie z. B. : Pyrrolin (z. B. 4,4-Dimethyl-pyrrolin), Oxazolin (z. B. 4,4-Dimethyloxazolin), Thiazolin (z. B. 5-Methylthiazolin), Selenazolin, Indolin (z. B. 3,3-Dimethylindolin, 3,3-Dimethyl-5-methoxyindolin, 3,3-Dimethyl-5-diethylaminoindolin), Benzimidazol (z. B. 1-Ethyl-5-trifluormethylbenzimidazol, 1-Methyl-5-chlorbenzimidazol, 1-Ethyl-5,6-dichlor-benzimidazol, 1-Ethyl-5-cyanbenzimidazol, 1-Methyl-5-carbethoxybenzimidazol, 1-Ethyl-5-acetylbenzimidazol, 1-Methyl-benzimidazol-5-sulfonsäurepyrrolidid, 1-Ethyl-benzimidazol-5-sulfonsäuredimethylamid, 1-Ethyl-5-phenylthiobenzimidazol, 1-Methyl-5-methylthiobenzimidazol, 1-Methyl-5-chlor-6-methyl-thiobenzimidazol), Oxazol (z. B. 4-Methyloxazol, 4,5-Diphenyloxazol, 4-Methyl-5-carbethoxyoxazol, Benzoxazol, 5-Chlorbenzoxazol, 5-Phenylbenzoxazol, 6-Methoxybenzoxazol, 5-Methoxybenzoxazol, 5-Methyl-6-methoxybenzoxazol, 5-Brombenzoxazol, 5-lodbenzoxazol, Naphtho-[2,1-d]-oxazol, Naphtho-[1,2-d]-oxazol, Naphtho-[2,3-d]-oxazol, 4,5,6,7-Tetrahydrobenzoxazol, Benzofuro-[2,3-f]-benzoxazol), Thiazol (z. B. 4-Methyl-thiazol, 4-Phenylthiazol, 4-Methyl-thiazol-5-acrylsäurethylester, Benzthiazol, 5-Methylbenzthiazol, 6-Methylbenzthiazol, 5-Chlorbenzthiazol, 5-Methoxybenzthiazol, 6-Methoxybenzthiazol, 5,6-Dimethylbenzthiazol, 5,6-Dimethoxybenzthiazol, 5-Methyl-6-methoxybenzthiazol, 5-Brombenzthiazol, 5-Phenylbenzthiazol, 6-Methylthiobenzthiazol, 6-Dimethylaminobenzthiazol, 5-Chlor-6-methoxybenzthiazol, 5,6-Methylendioxybenzthiazol, 6-β-Cyanethoxybenzthiazol, 5-Carbomethoxybenzthiazol, 5-Nitrobenzthiazol, 5-Phenylthiobenzthiazol, 5-Thienylbenzthiazol, 6-Hydroxybenzthiazol, 4,5,6,7-Tetrahydrobenzthiazol, 4-Oxo-4,5,6,7-tetrahydrobenzthiazol, Naphtho-[2,1-d]-thiazol, Naphtho-[1,2-d]-thiazol, 4,5-Dihydronaphtho-[1,2-d]-thiazol, 5-Methoxynaphtho-[1,2-d]-thiazol, 5,7,8-Trimethoxy-naphtho-[1,2-d]-thiazol), Selenazol (z. B. Benzselenazol, 5-Methylbenzselenazol, 5,6-Dimethylbenzselenazol, 5-Methoxybenzselenazol, 5-Methyl-6-methoxybenzselenazol, 5,6-Dimethoxy-benzselenazol, 5,6-Methylendioxybenzselenazol, 6-Methylbenzselenazol, Naphtho-[1,2-d]-selenazol), 1,2,3-Oxadiazol (z. B. 5-Methyl-1,3,4-oxadiazol, 5-Phenyl-1,3,4-oxadiazol), 1,3,4-Thiadiazol (z. B. 5-Methyl-1,3,4-thiadiazol, 2,5-Bis-methylthio-1,3,4-thiadiazol, 5-Benzylthio-1,3,4-thiazol, 2-Mercapto-5-methylthio-1,3,4-thiadiazol, 5-Carbethoxymethylthio-1,3,4-thiadiazol), Pyridin (z. B. 2-Methylpyridin, 4-Methylpyridin), Pyrimidin (z. B. 2-Methyl-4-methylthiopyrimidin), Chinolin (z. B. 6-Methylchinolin, 6-Methoxychinolin, 8-Chlorchinolin, 6-Fluorchinolin, 5,6-Benzochinolin, 6,7-Benzochinolin), Imidazol-[4,5-b]-chinoxalin.

m = 0 oder 1

Y = bedeutet Wasserstoff, Halogen, eine gesättigte oder ungesättigte aliphatische Gruppe, insbesondere mit bis zu 6 C-Atomen, gegebenenfalls substituiert, z. B. Methyl, Ethyl, Allyl, Cyanalkyl, Halogenalkyl, Alkoxyalkyl, Alkoxy, z. B. Carboxyalkoxy, Alkylthio, z. B. Carboxyalkylthio, Sulfoalkylthio, Carbalkoxyalkylthio, oder Mercapto.

Y = kann beispielsweise weiterhin eine Methinkette mit 1,3 oder 5 Methingruppen bedeuten, an deren Ende sich meist über die 2-Stellung angeknüpft eine N-alkylierte heterocyclische Base befindet, wie sie in

der Chemie der Cyaninfarbstoffe bekannt sind. Hierzu sei verwiesen auf F. M. Hamer, « The Cyanine Dyes and Ralated Compounds », (1964), Interscience Publishers John Wiley and Sons. Verbindungen der Formel V, in der Y in der erwähnten Weise definiert ist, werden als « entquaternierte Cyaninfarbstoffe » bezeichnet. Falls solche entquaternierte Cyaninfarbstoffe nach den Verfahren der Erfindung umgesetzt werden, sind die unmittelbaren Verfahrensprodukte ohne weitere Umsetzung als Sensibilisierungsfarbstoffe geeignet.

Die Umsetzungen werden im allgemeinen ohne Lösungsmittel durchgeführt, können jedoch auch in Gegenwart eines geeigneten Lösungsmittels vorgenommen werden. Als Lösungsmittel eignen sich alle in bezug auf die erfindungsgemäße Umsetzung inerten Lösungsmittel mit hohem Lösungsvermögen für die Reaktionspartner, wie z. B. Phenol oder m-Kresol sowie m-Xylol, Chlorbenzol, Anisol.

Die erfindungsgemäßen Umsetzungen können unter Abspaltung von Wasser erfolgen. In den meisten Fällen werden die erfindungsgemäßen Ester mit den Basen im Molverhältnis 1 : 2 umgesetzt, wobei pro Mol Base 0,5 Mol Wasser gebildet wird. Die Umsetzung kann aber auch in anderen Molverhältnissen — z. B. 1 : 1 — durchgeführt werden. Vorteilhafterweise wird das Wasser aus dem Reaktionsgefäß entfernt, beispielsweise durch

1) Arbeiten unter Vakuum,

2) Einleiten eines trockenen Inertgas, z. B. Stickstoff,

3) Gegenwart eines wasserentziehenden Mittels, entweder im Reaktionsgefäß (z. B. Anhydrid einer organischen Säure oder $P_4O_{10}$) oder in einer mit dem Reaktionsgefäß verbundenen Vorlage (z. B. $P_4O_{10}$, konzentrierte Schwefelsäure, NaOH oder andere Trockenmittel),

4) Verdampfen und Ausfrieren in einer Gefrierapparatur.

Die nach dem Verfahren der Erfindung hergestellten Sulfobetaine tertiärer Amine sind insbesondere solche der folgenden Formel VI

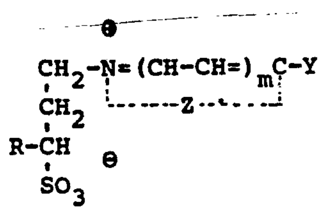

(VI)

worin Y, m, Z und R die bereits angegebene Beudeutung haben.

Diese Verbindungen finden unterschiedliche Verwendung. Sie sid beispielsweise als Leitsalze in der Galvanotechnik geeignet. Des weiteren handelt es sich bei diesen Verbindungen bei geeigneter Bedeutung von Y, d. h. wenn Y, wie bereits erwähnt, eine Methinkette mit 1, 3 oder 5 Methingruppen, an deren Ende sich eine N-alkylierte heterocyclische Base befindet, bedeutet, um die Endprodukte einer Cyaninfarbstoffsynthese. Derartige Verbindungen finden unmittelbar Verwendung für die spektrale Sensibilisierung von lichtempfindlichen Silberhalogenidemulsionen. Aber auch als Zwischenprodukte bei der Synthese von Polymethinfarbstoffen sind die nach dem Verfahren gemäß der Erfindung hergestellten Verbindungen wichtig. So werden beispielsweise die nach dem erfindungsgemäßen Verfahren hergestellten Sulfopropylquartärsalze heterocyclischer Basen mit Vorteil nicht isoliert, sondern nach Beendigung der Quaternierungsreaktion ohne weitere Reinigungsoperation in bekannter Weise weiter zu Polymethinfarbstoffen umgesetzt.

Die Verwendung der erfindungsgemäßen Sulfoalkylierungsmittel zur Sulfoalkylierung von tertiären Aminen wird im folgenden näher erläutert :

## Beispiel 3

Anhydro-1,2-dimethyl-3-[3-sulfopropyl]-5,6-dichlorbenzimidazoliumhydroxid, Fp. > 340 °C.

21,5 g 1,2-Dimethyl-5,6-dichlorbenzimidazol werden zusammen mit 14 g 3-Hydroxipropansulfonsäure-(3-sulfopropyl)-ester (im folgenden als « Ester 1 » bezeichnet) in 40 ml N-Methylpyrrolidon gelöst und 8 Stunden auf 190 °C Ölbadtemperatur unter schwachem Überleiten von Stickstoff erhitzt. Nach dem Abkühlen wird das Reaktionsprodukt mit 60 ml Ethanol verrieben und abgesaugt.

Ausbeute : 25,6 g = 76 % der Theorie

## Beispiel 4

Anhydro-1,2-dimethyl-5-cyan-3-(3-sulfopropyl)-benzimidazoliumhydroxid, Fp. > 345 °C.

23 g 1,2-Dimethyl-5-cyanbenzimidazol werden mit 16,3 g des Esters 1 analog Beispiel 3 auf 175 °C erhitzt. Aufarbeitung erfolgt mit 45 ml Methanol.

Ausbeute : 26,4 g = 69,2 %

Beispiel 5

Anhydro-1,2-dimethyl-5-pyrrolidinosulfonyl-3-(3-sulfopropyl)-benzimidazoliumhydroxid, Fp 327 °C (Zers.).

Analog Beispiel 3 — jedoch ohne Lösungsmittel — aus 16,8 g 1,2-Dimethyl-5-pyrrolidinosulfonylbenzimidazol und 10,9 g des Esters 1 in 4 Stunden bei 185 °C. Aufarbeitung mit Ethanol.
Ausbeute : 17,5 g = 73 %

Beispiel 6

Anhydro-2-methyl-3-(3-sulfopropyl)-naphtho-[1,2 : d]-oxazoliumhydroxid, Fp. 273-274 °C.

18,3 g 2-Methylnaphtho-[1,2 : d]-oxazol werden mit 14,2 g des Esters 1 und 7 g Phenol 6 Stunden im Ölbad auf 180 °C unter Stickstoff erhitzt. Aufarbeitung mit 30 ml Ethanol.
Ausbeute : 18,5 g = 61 %

Beispiel 7

Anhydro-2-methyl-3-(3-sulfopropyl)-benzthiazoliumhydroxid, Fp. 290 °.

7,5 g 2-Methylbenzthiazol und 6,6 g des Esters 1 werden zusammen. 5 Stunden auf 175 °C unter Überleiten von Stickstoff erhitzt. Nach Abkühlung wird mit 20 ml Ethanol aufgearbeitet.
Ausbeute : 9,3 g = 68 %

Beispiel 8

Anhydro-2-methyl-5-chlor-3-(3-sulfopropyl)-benzthiazoliumhydroxid, Fp. 288-290 °C.

a) 184 g 2-Methyl-5-chlor-benzthiazol werden mit 140 g des Esters 1 1,5 Stunden auf 175-180 °C erwärmt, dann werden 20 g Phenol in 20 ml Toluol gelöst zugegeben und weitere 4 Stunden im Stickstoffstrom erwärmt. nach Abkühlung wird mit 80 ml Ethanol aufgearbeitet.
Ausbeute : 276 g = 90 %

b) Die gleiche Ausbeute wird erhalten, wenn ohne Phenol und Toluol 5 Stunden auf 175 °C erhitzt wird.

Beispiel 9

Anhydro-2-methyl-3-(3-sulfobutyl)-benzthiazoliumhydroxid, Fp. 246 °C.

Aus 6 g 2-Methylbenzthiazol und 5,9 g 4-Hydroxibutan-2-sulfonsäure-(3-sulfobutyl)-ester (« Ester 2 ») durch 3,5-stündiges Erhitzen auf 175 °C und Aufarbeitung mit Isopropanol, dann Aceton.
Ausbeute : 5,9 g = 54 %

Beispiel 10

Anhydro-2-methyl-3-(3-sulfopropyl)-naphtho-[1,2 : d]-thiazoliumhydroxid, Fp. 277 °C.

a) 8 g des Esters 1 werden mit 5 ml Essigsäureanhydrid 2 Stunden auf 140 °C und nach Zusatz von 10 g 2-Methylnaphtho-[1,2 : d]-thiazol weitere 4 Stunden auf 175 °C erwärmt. Nach Aufarbeitung mit Ethanol werden 7,1 g = 44 % der Verbindung erhalten.

b) 50 g des Esters 1 werden im Rotationsverdampfer 4 Stunden im Vakuum von 20 mm Hg auf 140 °C erwärmt, wobei durch den Kühler eine Kühlflüssigkeit von − 20 °C gepumpt wird. Danach werden 50 g 2-Methyl-naphtho-[1,2 : d]-thiazol dazugegeben und weitere 4 Stunden auf 175 °C erwärmt. Das Reaktionsprodukt wird aus m-Kresol-Ethanol umkristallisiert.
Ausbeute : 75 g = 93 %

Beispiel 11

3-Ethyl-5-[3-(3-sulfopropyl)-2-benzthiazolyliden]-rhodanin, Na-Salz, Fp. 340 °C.

6,2 g 3-(Benzthiazol-2-ylthio)-propansulfonsaures Natrium werden mit 3 g des Esters 1 und 2 g Phenol 8,5 Stunden auf 175 °C unter Stickstoff erwärmt. Das Reaktionsprodukt wird in Ethanol aufgenommen, mit 3 g 3-Ethylrhodanin und 2 g Triethylamin versetzt und 3 Stunden gerührt. Der Farbstoff wird aus Methanol-Wasser umkristallisiert.

Ausbeute : 2,6 g = 25 %. Absorptionsmaximum : 427 nm.

Beispiel 12

Anhydro-2-methyl-3-(3-sulfopropyl)-5-methoxybenzselenazoliumhydroxid, Fp. 299-302 °C.

Durch 7,5 stündiges Erhitzen von 6,8 g 2-Methyl-5-methoxybenzselenazol mit 3,9 g des Esters 1 und 2 ml Acetanhydrid sowie 2 ml m-Kresol auf 175 °C, wobei während 1/2 Stunde ein schwacher Stickstoffstrom durchgeleitet wird, und Aufarbeitung mit Ethanol.
Ausbeute 6,8 g = 65 %

Beispiel 13

Anhydro-2-methyl-3-(3-sulfopropyl)-5-methyl-6-methoxy-benzselenazoliumhydroxid, Fp. 298-299 °C.

Analog Beispiel 12 aus 7,2 g 2,5-Dimethyl-6-methoxy-benzselenazol, 4 g Ester 1 und 3 g Phenol in 8 Stunden bei 175 °C.
Ausbeute : 7,7 g = 71 %

Beispiel 14

Anhydro-2-methyl-3-(3-sulfopropyl)-5-phenylbenzoxazoliumhydroxid, Fp. 288-290 °C.

Zu 11,2 g des Esters 1 werden 16,7 g 2-Methyl-5-phenylbenzoxazol und 5 g Phosphorpentoxid gegeben und die Mischung schwach — auf 30-40 °C — erwärmt. Daraufhin steigt die Temperatur von selbst auf 90° an. Dann wird unter schnellem Rühren noch 30 Minuten auf 175 °C (Ölbadtemperatur) erwärmt, wobei Kristallisation eintritt. Nach Abkühlung und mechanischer Zerkleinerung wird das Produkt mit 20 ml Methanol verrührt, abgesaugt und mit wenig kaltem Methanol gewaschen.
Ausbeute : 17,3 g = 65 %

Beispiel 15

3-Ethyl-5-[5-methylthio-3-(3-sulfopropyl)-1,3,4-thiadiazol-2-yliden]-rhodanin, Pyridinsalz, Fp. 203-204 °C.

1,8 g 2,5-Bis-methylthio-1,3,4-thiadiazol werden mit 2,8 g des Esters 1 unter Zusatz von 0,5 g Phenol 2 Stunden auf 140 °C unter Rühren erwärmt. Das Reaktionsgemisch wird auf 80 °C abgekühlt, mit 1,6 g 3-Ethylrhodanin und 5 ml Pyridin versetzt und 5 Minuten bei 80 °C gehalten. Man läßt den Farbstoff 2 Stunden kristallisieren, saugt ab und kristallisiert aus 10 ml n-Propanol um.
Ausbeute : 1,2 g = 24,4 %. Absorptionsmaximum : 420 nm.

Beispiel 16

3,3'-Bis-[3-sulfopropyl]-4,5 ; 4', 5'-dibenzothiacyanin, Triethylaminsalz, Fp. 291 °C.

22 g 2-Mercaptonaphtho-[1,2 : d]-thiazol, 30 g des Esters 1 und 5 g Phenol werden bei 175 °C verschmolzen und 6 Stunden unter Überleiten von Stickstoff bei 175 °C gerührt. Nach Abkühlung auf 100 °C wird das Reaktionsprodukt in 60 g Phenol gelöst und eine Schmelze von 32 g Anhydro-2-methyl-3-[3-sulfopropyl]-naphtho-[1,2 : d]-thiazoliumhydroxid in 60 g Phenol dazugegeben. nach Abkühlung auf 50-60 °C werden unter Rühren 34 ml Triethylamin dazugetropft. Man läßt noch 10 Minuten reagieren und gibt 300 ml auf 50 °C erwärmtes Acetonitril hinzu. Nach 4 stündigem Stehen wird der abgeschiedene Farbstoff abgesaugt und aus Methylenchlorid-Methanol umkristallisiert.
Ausbeute : 15 g. Absorptionsmaximum : 455 nm.

**Ansprüche**

1. Hydroxyalkansulfonsäure-(sulfoalkyl)-ester der Formel

$$HO-(\underset{R}{CH})_nSO_2-O-(\underset{R}{CH})_n-SO_3X$$

worin bedeuten
X = $H^+$ oder ein Kation,
n = 3 oder 4,

und die Reste R in jeder Gruppe $-\underset{R}{C}H-$ unabhängig voneinander H oder Alkyl mit bis zu 4 C-Atomen.

2. Hydroxypropansulfonsäure-(sulfopropyl)-ester der folgenden Formel

$$HO(CH_2)_2\text{-}\underset{R}{CH}\text{-}SO_2\text{-}O(CH_2)_2\text{-}\underset{R}{CH}\text{-}SO_3X$$

worin bedeuten

R = H oder ein Alkyl mit bis zu 4 C-Atomen,

X = $H^+$ oder ein Kation.

3. Hydroxybutansulfonsäure-(sulfobutyl)-ester der folgenden Formel

$$HO(CH_2)_3\text{-}\underset{R}{CH}\text{-}SO_2\text{-}O(CH_2)_3\text{-}\underset{R}{CH}\text{-}SO_3X$$

worin bedeuten

R = H oder ein Alkyl mit bis zu 4 C-Atomen,

X = $H^+$ oder ein Kation.

4. Verbindungen nach den Ansprüchen 1-3, dadurch gekennzeichnet, daß R = Wasserstoff oder methyl bedeutet.

5. Verfahren zur Herstellung von Hydroxyalkansulfonsäure (sulfoalkyl)-ester, dadurch gekennzeichnet, daß eine Hydroxyalkansulfonsäure des Propans oder Butans oder ein Alkansulton mit 5 oder 6 Ringgliedern mit der 1- bis 4-fachen molaren Menge Wasser auf Temperaturen zwischen 80 und 180 °C erhitzt wird.

6. Verfahren nach Anspruch 5, dadurch gekennzeichnet, daß das Verfahren bei Temperaturen zwischen 120 und 150 °C durchgeführt wird.

7. Sulfoalkylierungsmittel, herstellbar durch Erhitzen von 3-Hydroxypropansulfonsäure oder 1,3-Propansulton mit der 1- bis 4-fachen molaren Menge Wasser auf Temperaturen zwischen 80 und 180 °C, gekennzeichnet durch folgende $^{13}C$-NMR-shifts (ppm, relativ zu TMS = 0, in $D_2O$ in Gegenwart von Dioxan) : 25,2 ; 27,8 ; 48,8 ; 61,1 ; 69,5.

8. Sulfoalkylierungsmittel, herstellbar durch Erhitzen von 4-Hydroxybutan-2-sulfonsäure oder 3-Methyl-1,3-propansulton (= 3-Methyl-1,2-oxathiolan-2,2-dioxid) mit der 1- bis 4-fachen molaren Menge Wasser auf Temperaturen zwischen 80 und 180 °C, gekennzeichnet durch folgende $^{13}C$-NMR-shifts (ppm, relativ zu TMS = 0, in $D_2O$ in Gegenwart von Dioxan) : 15,4 ; 31,7 ; 34,4 ; 53,6 ; 60,0 ; 68,5.

9. Verfahren zur Herstellung von Sulfoalkylquartärsalzen tertiärer Amine durch Umsetzung eines tertiären Amins mit einem Sulfoalkylierungsmittel bei einer Temperature zwischen 100 und 250 °C, dadurch gekennzeichnet, daß als Sulfoalkylierungsmittel ein Sulfoalkylierungsmittel nach einem der Ansprüche 1-4, 7 oder 8 verwendet wird.

10. Verfahren nach Anspruch 9, dadurch gekennzeichnet, daß ein tertiäres Amin der folgenden Formel umgesetzt wird

```
N=(CH-CH)=)mC-Y
:            :
:....Z.......:
```

worin bedeuten

Y Wasserstoff ; Halogen, eine gesättigte oder ungesättigte aliphatische Gruppe mit bis zu 6 Kohlenstoffatomen ; Alkoxy ; Alkylthio oder Mercapto

Z die zur Vervollständigung einer heterocyclischen Gruppe mit mindestens einem 5- oder 6-gliedrigen heterocyclischen Ring erforderlichen Atome

m 0 oder 1.

11. Verfahren nach den Ansprüchen 9 oder 10, dadurch gekennzeichnet, daß die Umsetzung bei einer Temperatur zwischen 140 und 200 °C durchgeführt wird.

12. Verfahren nach Anspruch 10, dadurch gekennzeichnet, daß die Umsetzung in Gegenwart eines Lösungsmittels durchgeführt wird.

13. Verfahren nach Anspruch 10, dadurch gekennzeichnet, daß die Umsetzung in Gegenwart eines Phenols durchgeführt wird.

14. Verfahren nach Anspruch 10, dadurch gekennzeichnet, daß die Umsetzung in Gegenwart eines Anhydrids einer organischen Säure durchgeführt wird.

15. Verfahren nach Anspruch 10, dadurch gekennzeichnet, daß die Umsetzung in Gegenwart von Phosphorpentoxid durchgeführt wird.

16. Verfahren nach Anspruch 10, dadurch gekennzeichnet, daß das Reaktionsprodukt ohne Isolierung in bekannter Weise zu einem Cyaninfarbstoff weiter umgesetzt wird.

**Claims**

1. Hydroxy alkane sulphonic acid (sulphoalkyl) ester of the formula

$$HO-(\underset{R}{CH})_n SO_2-O-(\underset{R}{CH})_n-SO_3X$$

wherein
X denotes $H^+$ or a cation,
n denotes 3 or 4,
and the radicals R in each $-\underset{R}{CH}-$ group independently of one another denote H or alkyl with up to 4 C atoms.

2. Hydroxy propane sulphonic acid (sulphopropyl) ester of the following formula

$$HO(CH_2)_2-\underset{R}{CH}-SO_2-O(CH_2)_2-\underset{R}{CH}-SO_3X$$

wherein
R denotes H or an alkyl with up to 4 C atoms, and
X denotes $H^+$ or a cation.

3. Hydroxy butane sulphonic acid (sulphobutyl) ester of the following formula

$$HO(CH_2)_3-\underset{R}{CH}-SO_2-O(CH_2)_3-\underset{R}{CH}-SO_3X$$

wherein
R denotes H or an alkyl with up to 4 C atoms, and
X denotes $H^+$ or a cation.

4. Compounds according to Claims 1-3, characterised in that R denotes hydrogen or methyl.

5. Process for the preparation of hydroxy alkane sulphonic acid (sulphoalkyl) ester, characterised in that a hydroxy alkane sulphonic acid of propane or butane or an alkane sultone having 5 or 6 ring members is heated to temperatures between 80 and 180 °C with 1 to 4 times the molar amount of water.

6. Process according to Claim 5, characterised in that the process is carried out at temperatures of between 120 and 150 °C.

7. Sulphoalkylating agent, obtainable by heating 3-hydroxypropane sulphonic acid or 1,3-propane sultone with 1 to 4 times the molar amount of water to temperatures of between 80 and 180 °C, characterised by the following $^{13}C$ NMR shifts (ppm, relative to TMS = 0, in $D_2O$ in the presence of dioxane) : 25.2 ; 27.8 ; 48.8 ; 61.1 ; 69.5.

8. Sulphoalkylating agent, obtainable by heating 4-hydroxy-butane-2-sulphonic acid or 3-methyl-1,3-propane sultone (= 3-methyl-1,2-oxathiolane-2,2-dioxide) with 1 to 4 times the molar amount of water to temperatures of between 80 and 180 °C, characterised by the following $^{13}C$ NMR shifts (ppm, relative to TMS = 0, in $D_2O$ in the presence of dioxane) : 15.4 ; 31.7 ; 34.4 ; 53.6 ; 60.0 ; 68.5.

9. Process for the preparation of sulphoalkyl quaternary salts of tertiary amines by reacting a tertiary amine with a sulphoalkylating agent at a temperature of between 100 and 250 °C, characterised in that the sulphoalkylating agent used is a sulphoalkylating agent according to one of Claims 1-4, 7 or 8.

10. Process according to Claim 9, characterised in that a teriary amine of the following formula is reacted

```
N=(CH-CH)=)mC-Y
:            :
:....Z.......:
```

wherein
Y denotes hydrogen ; halogen, or a saturated or unsaturated aliphatic group with up to 6 carbon atoms ; alkoxy ; alkylthio or mercapto,
Z denotes the atoms required to complete a heterocyclic group containing at least one 5- or 6-membered heterocyclic ring and
m denotes 0 or 1.

11. Process according to Claims 9 or 10, characterised in that the reaction is carried out at a temperature of between 140 and 200 °C.

12. Process according to Claim 10, characterised in that the reaction is carried out in the presence of a solvent.

13. Process according to Claim 10, characterised in that the reaction is carried out in the presence of a phenol.

14. Process according to Claim 10, characterised in that the reaction is carried out in the presence of an anhydride of an organic acid.

15. Process according to Claim 10, characterised in that the reaction is carried out in the presence of phosphorus pentoxide.

16. Process according to Claim 10, characterised in that the reaction product, without being isolated, is further reacted in a known manner to give a cyanine dyestuff.

**Revendications**

1. Hydroxyalcanesulfonates de sulfoalkyles de formule

$$HO-(\underset{R}{CH})_nSO_2-O-(\underset{R}{CH})_n-SO_3X$$

dans laquelle

X = H+ ou un cation,

n = 3 ou 4,

et les restes R dans chaque groupe $-\underset{R}{CH}-$ représentent indépendamment l'un de l'autre H ou un alkyle jusqu'en $C_4$.

2. Hydroxypropanesulfonates de sulfopropyles de formule suivante

$$HO(CH_2)_2-\underset{R}{CH}-SO_2-O(CH_2)_2-\underset{R}{CH}-SO_3X$$

dans laquelle

R = H ou un alkyle jusqu'en $C_4$,

X = $H^+$ ou un cation.

3. Hydroxybutanesulfonates de sulfobutyles de formule suivante

$$HO(CH_2)_3-\underset{R}{CH}-SO_2-O(CH_2)_3-\underset{R}{CH}-SO_3X$$

dans laquelle

R = H ou un alkyle jusqu'en $C_4$,

X = $H^+$ ou un cation.

4. Composés selon les revendications 1-3, caractérisé en ce que R représente l'hydrogène ou un groupe méthyle.

5. Procédé pour la fabrication d'hydroxyalcanesulfonates de sulfoalkyles, caractérisé en ce qu'on chauffe un acide hydroxyalcanesulfonique du propane ou du butane ou une alcanesultone à 5 ou 6 chaînons avec la quantité 1 à 4 fois molaire d'eau à des températures entre 80 et 180 °C.

6. Procédé selon la revendication 5, caractérisé en ce que le procédé est mis en œuvre à des températures entre 120 et 150 °C.

7. Agent de sulfoalkylation, pouvant être fabriqué par chauffage d'acide 3-hydroxypropanesulfonique ou de 1,3-propanesultone avec la quantité 1 à 4 fois molaire d'eau à des températures entre 80 et 180 °C, caractérisé par les déplacements de RMN de $^{13}C$ suivants )ppm, par rapport au TMS = 0, dans $D_2O$, en présence de dioxanne) : 25,2 ; 27,8 ; 48,8 ; 61,1 ; 69,5.

8. Agent de sulfoalkylation, pouvant être fabriqué par chauffage d'acide 4-hydroxybutane-2-sulfonique ou de 3-méthyl-1,3-propanesultone (= 3-méthyl-1,2-oxathiolanne-2,2-dioxyde) avec la quantité 1 à 4 fois molaire d'eau à des températures entre 80 et 180 °C, caractérisé par les déplacements de RMN de $^{13}C$ suivants )ppm, par rapport au TMS = 0, dans $D_2O$, en présence de dioxanne) : 15,4 ; 31,7 ; 34,4 ; 53,6 ; 60,0 ; 68,5.

9. Procédé pour la fabrication de sels sulfoalkylquaternaires d'amines tertiaires par réaction d'une amine tertiaire avec un agent de sulfoalkylation à une température entre 100 et 250 °C, caractérisé en ce que l'on utilise comme agent de sulfoalkylation un agent de sulfoalkylation selon l'une des revendications 1-4, 7 ou 8.

10. Procédé selon la revendication 9, caractérisé en ce que l'on fait réagir une amine tertiaire de formule suivante :

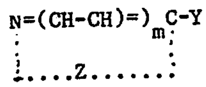

dans laquelle

Y représente l'hydrogène, un halogène ou un groupe aliphatique saturé ou insaturé jusqu'en $C_6$, alcoxy, alkylthio ou mercapto,

Z représente les atomes nécessaires pour compléter un groupe hétérocyclique ayant au moins un noyau hétérocyclique à 5 ou 6 chaînons, et

m est égal à 0 ou 1.

11. Procédé selon les revendications 9 ou 10, caractérisé en ce que la réaction est effectuée à une température entre 140 et 200 °C.

12. Procédé selon la revendication 10, caractérisé en ce que la réaction est effectuée en présence d'un solvant.

13. Procédé selon la revendication 10, caractérisé en ce que la réaction est effectuée en présence d'un phénol.

14. Procédé selon la revendication 10, caractérisé en ce que la réaction est effectuée en présence d'un anhydride d'un acide organique.

15. Procédé selon la revendication 10, caractérisé en ce que la réaction est effectuée en présence de pentoxyde de phosphore.

16. Procédé selon la revendication 10, caractérisé en ce que l'on fait encore réagir sans isolement le produit de réaction de manière connue en un colorant de cyanine.